# EUROPÄISCHE PATENTSCHRIFT

(11) **EP 2 866 727 B1**
(45) Veröffentlichungstag und Bekanntmachung des Hinweises auf die Patenterteilung: **06.07.2016**
(21) Anmeldenummer: 13721906.9
(22) Anmeldetag: 26.04.2013
(51) Int. Cl.: A61F 2/00

(54) **INKONTINENZIMPLANTAT**
INCONTINENCE IMPLANT
IMPLANT POUR INCONTINENCE

(30) Priorität: 29.06.2012 DE 202012006290 U
(43) Veröffentlichungstag der Anmeldung: 06.05.2015
(73) Patentinhaber: Pregenzer, Lukas, 6414 Mieming (AT)
(72) Erfinder: PREGENZER, Lukas, 6414 Mieming (AT); PREGENZER, Bruno, 6414 Mieming (AT); STENZEL, Arnulf, 72076 Tübingen (DE)
(74) Vertreter: Laufhütte, Dieter
(86) Internationale Anmeldenummer: PCT/EP2013/001264
(87) Internationale Veröffentlichungsnummer: WO 2014/000838

(56) Entgegenhaltungen:
- EP-B1- 1 154 732
- US-A1- 2008 073 903

## Beschreibung

Die Erfindung betrifft einen implantierbaren, zwischen zwei stabilen Stellungen schaltbaren Betätigungsmechanismus für ein zwischen zwei Zuständen oder Stellungen schaltbares Implantat, mit einem Stützelement, das in einer Montageplatte zur Abstützung an einen Knochen aufweist und mit einem relativ zum Stützelement bewegbaren, federbeaufschlagten Betätigungselement, das sich durch das Stützelement erstreckt und mittels eines Seils mit einem einen Wechsel zwischen den beiden Stellungen des Implantates auslösenden, jenseits des Stützelementes vorgesehenen Aktivierungsteil verbunden ist.

Zur Verhinderung der Harninkontinenz ist aus der EP-A-639 355 eine die Harnröhre untergreifende Bandschlaufe aus einem körperverträglichen Fremdmaterial vorgesehen, wobei die Bandenden an einer höheren Stelle im Körper fixiert werden, und der Mittelbereich der Bandschleife eine mit einem Fluid füllbare Kammer darstellt, wobei die Menge des eingefüllten Fluids die Auflagehöhe der Harnröhre bestimmt und daher ebenfalls justiert werden kann. In der EP-A 639 355 ist auch angeführt, dass schon Faszien für die Bildung einer Bandschlaufe verwendet worden sind, die im Körper fixiert werden. Eine spätere Korrektur aufgrund von Veränderungen ist allerdings kaum möglich.

Einen weiteren implantierbaren Betätigungsmechanismus zeigt die US 5,518,504 A. Hier wird eine die Harnröhre untergreifende Bandschlaufe ebenfalls aus einer hochgezogenen Ruhestellung mittels eines hydraulischen Systems abgesenkt, das einen Kolbenpumpenpaar umfasst.

Die Nachteile dieser Einrichtung liegen in der Verwendung eines Druckfluids und der zu verlegenden Schläuche und der Gefahr des Leckwerdens bei der Notwendigkeit eines zu implantierenden Vorratsbehälters. Ein Betätigungsmechanismus der eingangs genannten Art ist für eine Absperreinrichtung für natürliche, röhrenartige Körperorgane beispielsweise aus der EP 1 154 732 B1 zu entnehmen. Hier ist bereits ein implantierbarer Betätigungsmechanismus geschaffen worden, der ohne hydraulisches System ein zwischen zwei Zuständen oder Stellungen schaltbares Implantat betätigt, und insbesondere eine Harninkontinenz verhindert. Bei diesen vorbekannten Betätigungsmechanismus ist allerdings ein Betätigungselement eingesetzt worden, in welcher eine Führungshülse mit einer herzförmigen Führungsbahn vorgesehen ist, entlang der ein einzelner Führungsstift entlanggleitet und hierdurch unter Federlast zum jeweiligen Einrasten in einer definierten Rastposition des Betätigungselementes führt. Es hat sich gezeigt, dass dieser Rastmechanismus einer Dauerbelastung nicht standhält.

Aufgabe der Erfindung ist es daher, einen gattungsgemäßen implantierbaren Betätigungsmechanismus derart weiterzubilden, dass er auch im Dauerbetrieb trotz langen Betriebszeiten präzise arbeitet.

Erfindungsgemäß wird diese Aufgabe durch die Kombination der Merkmale des Anspruchs 1 gelöst.

Demzufolge ist ein implantierbarer, zwischen zwei stabilen Stellungen schaltbarer Betätigungsmechanismus für ein zwischen Zuständen oder Stellungen schaltbares Implantat geschaffen, bei dem das federbeaufschlagte Betätigungselement einen Führungsring mit mehr als einem Fortsatz aufweist, wobei die Fortsätze derart in einer Führungskulisse geführt sind, dass sie unter Federlast in zwei die vorgenannten Stellungen definierenden Rastpositionen einrastbar sind.

Erfindungsgemäß wird also das vorbekannte eindimensionale Führungsteil durch ein mehrdimensionales Führungsteil ersetzt. Hierdurch kann der Verschleiß aufgrund der Dauerbelastung erheblich reduziert werden, so dass der implantierbare Betätigungsmechanismus eine wesentlich längere Nutzungsdauer gewährleistet, ohne dass ein erneuter Eingriff beim Patienten vorgesehen werden müsste.

Bevorzugte Ausgestaltungen des Betätigungsmechanismus ergeben sich aus den sich an den Hauptanspruch anschließenden Unteransprüchen.

Demnach ist der Führungsring vorteilhaft mit einer Führungshülse des Betätigungselementes verbunden. Die Führungskulisse dagegen kann in einer inneren Hülse des Betätigungselementes oder in einer eigenen mit dieser verbundenen Zwischenhülse vorgesehen sein.

Die Fortsätze können entweder auf der Innenseite des Führungsrings angeordnet oder aber in einer konstruktiven Umkehr auf der Außenseite des jeweiligen Führungsrings. Die Fortsätze weisen einen im Wesentlichen dreieckigen Querschnitt auf. Die Fortsätze sind jeweils so am Führungsring angeordnet, dass sie im Flächenkontakt mit der Führungskulisse stehen, in der sie eingreifen. Hierdurch wird die Abnutzung einzelner Elemente vermieden.

Besonders vorteilhaft ist der Führungsring über ein auf die Führungshülse aufschnappbares Schnappelement längsverschieblich auf der Führungshülse montierbar. Hierdurch ist eine besonders einfache und kosteneffiziente Montage möglich.

Eine besonders verschleißminimierte Ausführungsform des Führungsrings weist vier Fortsätze auf, die vorteilhaft gleich verteilt auf dem Umfang des Führungsrings angeordnet sind.

Anhand der nachstehenden Zeichnung werden weitere Einzelheiten, Merkmale und Vorteile der Erfindung anhand eines Ausführungsbeispiels näher erläutert. Es zeigen:
- Figur 1:: ein Längsschnitt durch einen Betätigungsmechanismus nach dem Stand der Technik in einer ersten Stellung,
- Figur 2:: ein Längsschnitt durch den Betätigungsmechanismus gemäß Figur 1 in einer zweiten Stellung,
- Figur 3:: eine Einbaustellung des Betätigungsmechanismus,
- Figur 4:: eine seitliche Darstellung eines Teils des erfindungsgemäßen Betätigungsmechanismus gemäß einer ersten Ausführungsvariante
- Figur 5:: ein Schnitt durch die Vorrichtung gemäß Figur 4,
- Figur 6:: eine seitliche Darstellung der Vorrichtung gemäß Figur 4 in einer anderen Stellung,
- Figur 7:: eine Schnittdarstellung durch die Figur 6,
- Figur 8:: eine perspektivische Explosionsdarstellung des Bauteils gemäß Figur 4,
- Figur 9:: eine Detaildarstellung eines Teils gemäß Figur 8,
- Figur 10:: eine perspektivische Detaildarstellung eines Teils gemäß Figur 8,
- Figur 11:: eine seitliche Darstellung eines Teils des erfindungsgemäßen Betätigungsmechanismus gemäß einer anderen Ausführungsvariante,
- Figur 12:: eine Schnittdarstellung durch die Vorrichtung gemäß Figur 11,
- Figur 13:: eine seitliche Darstellung der Vorrichtung gemäß Figur 11 in einer anderen Stellung,
- Figur 14:: eine Schnittdarstellung durch die Figur 13,
- Figur 15:: eine perspektivische Explosionsdarstellung des Bauteils gemäß Figur 11,
- Figur 16:: eine Detaildarstellung eines Teils gemäß Figur 15 und
- Figur 17:: eine perspektivische Detaildarstellung eines Teils gemäß Figur 15.

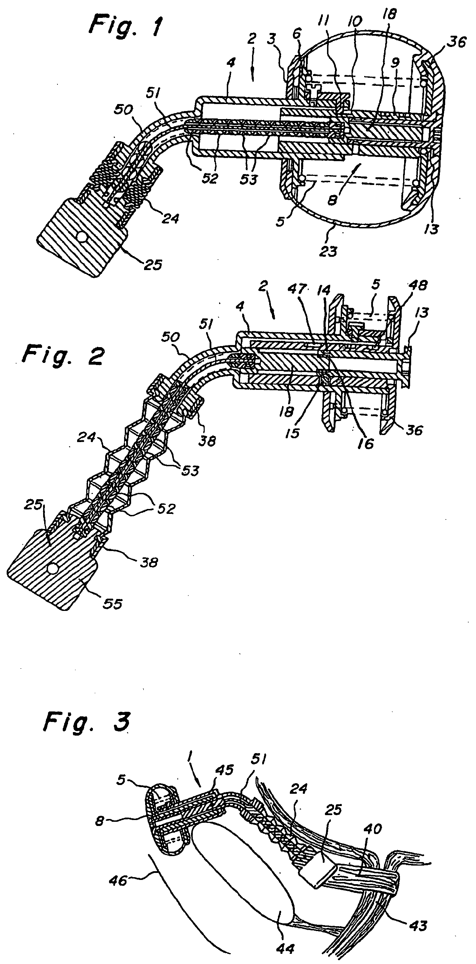

In den Figuren 1 bis 3 ist ein Betätigungsmechanismus nach dem Stand der Technik dargestellt, wie er insbesondere in der EP 1 154 732 A im Detail beschrieben ist, wobei ergänzend zu den nachfolgenden Ausführungen auf die dortige Beschreibung verwiesen wird. Der in den Figuren 1 bis 3 in seiner Gesamtheit dargestellte Betätigungsmechanismus dient zur Schaltung eines zwei stabile Schaltstellungen aufweisenden Implantates, insbesondere eines künstlichen Verschlusses eines Körperdurchgangs, einer Körperöffnung oder dergleichen, wie beispielsweise der Harnröhre (vgl. Figur 3). Der Betätigungsmechanismus 1 ist ebenfalls an geeigneter Stelle implantierbar und wird im Falle eines künstlichen Harnröhrenverschlusses am Schambein 44 bzw. der Symphyse abgestützt.

Der Betätigungsmechanismus 1 weist ein Stützelement 2 auf, das mit einer Montageplatte 3 und einer von einem Hüllrohr 45 umgebenen Führungshülse versehen ist. Das Stützelement 2 weist einen gebogenen Endabschnitt 50 auf, wodurch der Betätigungsmechanismus 1, wie Figur 3 zeigt, oberhalb des Schambeines angeordnet werden kann. Die Montageplatte 3 ist mit seitlichen senkrecht zur Zeichenebene abstehenden Befestigungslaschen versehen, die mit Knochenschrauben oder dergleichen am Schambein festlegbar sind.

Der Montageplatte 3 ist weiters eine Klemmplatte 6 zugeordnet, wobei zwischen der Montageplatte 3 und der Klemmplatte 6 ein Faltenbalk bzw. Ballon 23 aus einem physiologisch verträglichen Kunststoff oder dergleichen dichtend eingeklemmt ist, dessen anderer Rand an einem Flansch 36 eines Betätigungselementes 8 dichtend gehalten ist, das in der Führungshülse 4 des Stützelementes 2 verschiebbar geführt ist. Das Betätigungselement 8 weist eine innere Hülse 9 und eine Führungshülse 4 auf, zwischen denen eine Rückstellfeder 5 angeordnet ist, die das Betätigungselement 8 in die in Figur 1 gezeigte Stellung beaufschlagt, in der das Betätigungselement 8 zum Stützelement hin bewegbar ist und die Aufnahme 25 den geringsten Abstand zur Führungshülse 4 aufweist. Der Faltenbalk 23 ist auseinandergezogen und ein zweiter Faltenbalk 24 zwischen zwei Überwurfmuttern 38 der Aufnahme 25 und der Führungshülse 4 stark gefaltet.

Innerhalb des Faltenbalkes 24 bzw. in seiner Wandung ist eine Rückstellfeder vorgesehen, die der innerhalb des ersten Faltenbalkes 23 angeordneten Rückstellfeder 5 entgegenwirkt, aber schwächer als diese ist.

In der Ausführungsform gemäß der Figuren 1 bis 3, die grundsätzlich schon bekannt ist, ist die Führungshülse 4 verschiebbar in der inneren Hülse 9 geführt. Diese ist, wie in der EP 1 154 732 A beschrieben ist, derart ausgestaltet, dass das Betätigungselement 8 zwei stabile Stellungen einnehmen kann. Die Figur 2 zeigt eine Stellung, nämlich die maximal komprimierte Stellung des Betätigungselementes 8, wobei hier der in der Figur 1 etwa ballonartig ausgebildete erste Faltenbalk 23 der Einfachheit halber nicht dargestellt ist. In der Darstellung gemäß Figur 2 wird der hier nicht näher dargestellte Faltenbalk 23 stark gefaltet, während der Faltenbalk 24, wie hier dargestellt, weit auseinandergezogen ist und die am Ende eines Seilzugs angeordnete Aufnahme 25 vom Ende der Führungshülse 4 einen großen Abstand aufweist. Von der in Figur 2 dargestellten Stellung kann das Betätigungselement 8 in die erste stabile Stellung zurückgeführt werden. Hier ist die Aufnahme 25 wieder an die Führungshülse 4 angenähert und der Faltenbalk 24 zusammengefaltet.

Das Seil 51 kann direkt in einen gebogenen Endabschnitt 50 geführt sein, oder aber wie in der Figur 1 und 2 dargestellt, in einer Seilhülle angeordnet sein, wenn beispielsweise zwischen den für das Seil 51 und den gebogenen Endabschnitten 50 verwendeten Materialien eine zu hohe Reibung gegeben wäre. Da die Seilhülle möglichst flexibel sein muss, um mit dem Seil 51 durch den gebogenen Endabschnitt 50 zu gleiten, ist die Seilhülle 51 bevorzugt aus aneinandergereihten Kugeln 52 und Zwischenscheiben 53 zusammengesetzt, die jeweils zwei konkave Anlageflächen für die Kugeln 52 aufweisen. Die Zwischenscheiben 53 sind dadurch auf den Kugeln 52 begrenzt verschwenkbar angeordnet, und der Seilzug kann durch den gebogenen Endabschnitt bewegt werden, wie der Vergleich der Figuren 1 und 2 zeigt.

Figur 3 zeigt eine bevorzugte Anwendung des Betätigungsmechanismus 1 zur Senkung eines operativ angehobenen Harnröhrenansatzes, d. h. zum künstlichen Öffnen und Verschließen einer Harnröhre 43 nahe der Harnblase. Als künstliches Verschlusselement dient eine Faszie 40, die unterhalb der Harnröhre 43 angeordnet wird und deren beiden Enden in der Aufnahme 25 des Betätigungselementes 8 fixiert werden.

In der ersten stabilen Stellung der implantierten, die Harnröhre 43 untergreifenden Faszie 40 ist die Harnröhre angehoben und so geknickt, dass ein Harnabfluss nicht möglich ist.

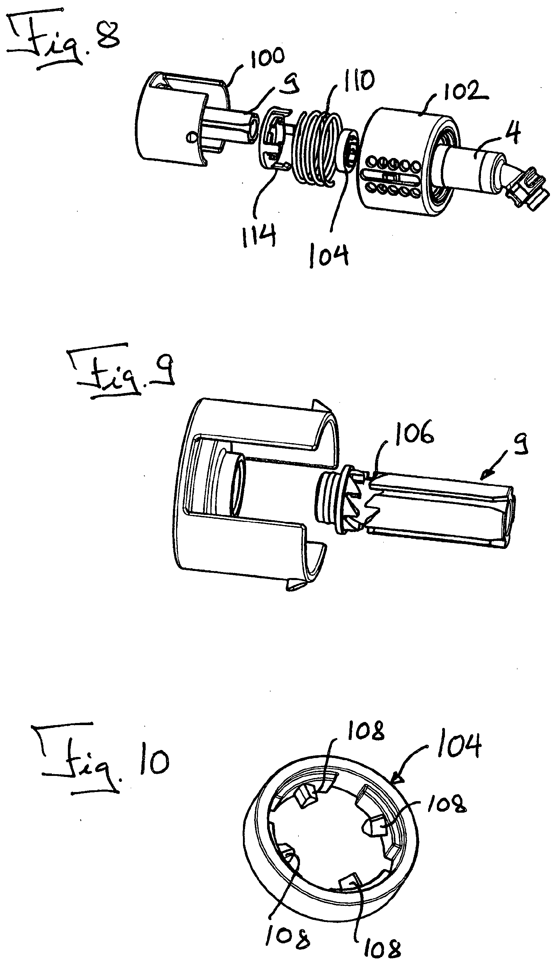

Wenn das Betätigungselement 8 durch äußere Druckeinwirkung auf eine unter der Haut 46 liegende Druckfläche in die in Figur 8 gezeigte Stellung überführt wird, erhöht sich der Abstand der Aufnahme 25 und die Faszie 40 wird um diese Differenz freigegeben, so dass der Harnröhrenansatz abgesenkt und die Harnröhre 43 geöffnet wird. Dies stellt die zweite stabile Stellung des Implantats dar (vgl. Figur 3). Eine nochmalige äußere Druckeinwirkung auf die Druckfläche löst die Rückstellung in die erstgenannte Position aus.

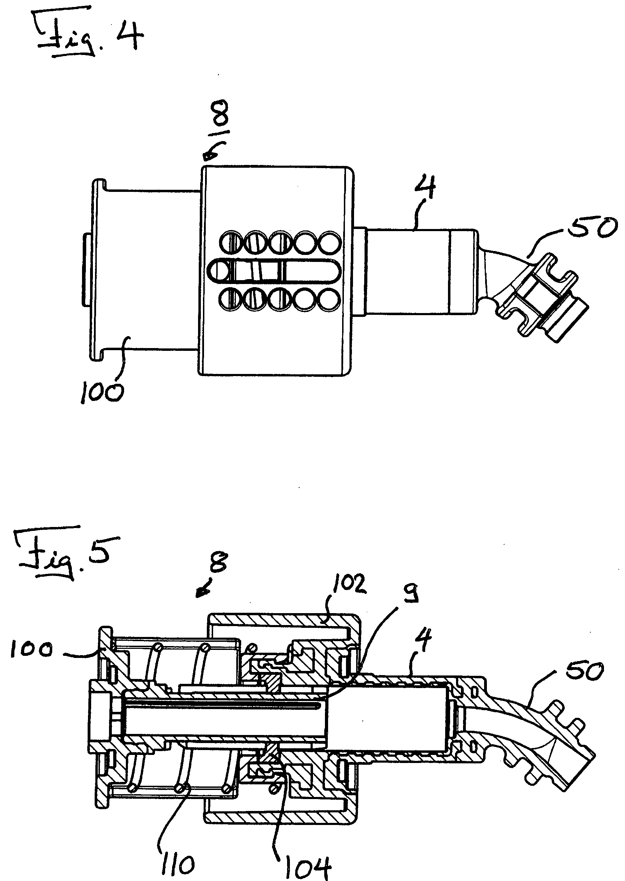

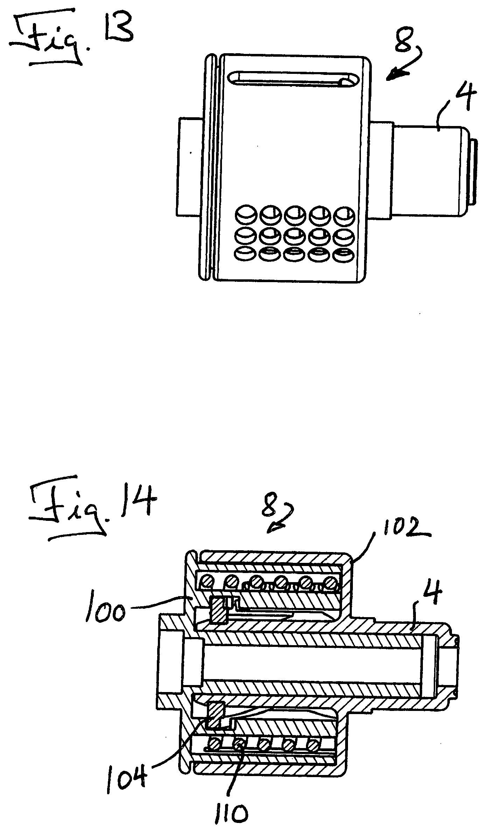

Anhand der Figuren 4 bis 10 und 11 bis 17 werden zwei Ausführungsbeispiele für die Ausführung des erfindungsgemäßen Betätigungsmechanismus 1 gezeigt. In dem erfindungsgemäßen Betätigungsmechanismus ist jeweils das Betätigungselement 8 gemäß der Figuren 1 bis 3 neu gestaltet worden. Die übrigen Teile des Betätigungsmechanismus 1, wie sie in den Figuren 1 bis 3 dargestellt sind, werden in hier nicht näher dargestellter Art und Weise in gleicher Weise ausgeführt, wie dies anhand der Figuren 1 bis 3 zuvor beschrieben wurde.

In der anhand der Figuren 4 bis 10 dargestellten Ausführungsvariante ist zunächst die Führungshülse 4 zu erkennen, die derjenigen gemäß der Figuren 1 und 2 entspricht. An diese schließt sich der gebogene Endabschnitt 50 an, der ebenfalls der Ausführungsform gemäß der Figuren 1 bis 3 entspricht. Das Betätigungselement 8 weist, wie in Figur 5 dargestellt, eine in der Führungshülse 4 verschiebbare innere Hülse 9 auf.

An dem freien Ende der inneren verschiebbaren Hülse 9 ist ein topfförmiges Hülsenelement 100 angeordnet. Dieses topfförmige Hülsenelement 100 korrespondiert mit einem weiteren topfförmigen Hülsenelement 102, das in der gemäß Figur 5 ersichtlichen Art und Weise mit der Führungshülse 4 verbunden ist. Mit dem topfförmigen Hülsenelement 102 ist weiterhin ein Führungsring 104 verbunden, der mit einer Führungskulisse 106 zusammenwirkt, die in der Hülse 9 vorgesehen ist. Diesbezüglich wird auf die Darstellung gemäß Figur 9 verwiesen, in der die entsprechende Führungsnut 106 für den Führungsring 104 im Detail zu erkennen ist. Der Führungsring 104 ist in besonders einfacher Weise über ein aufschnappbares Schnappelement 114 längsverschieblich auf der Führungshülse 4 montiert.

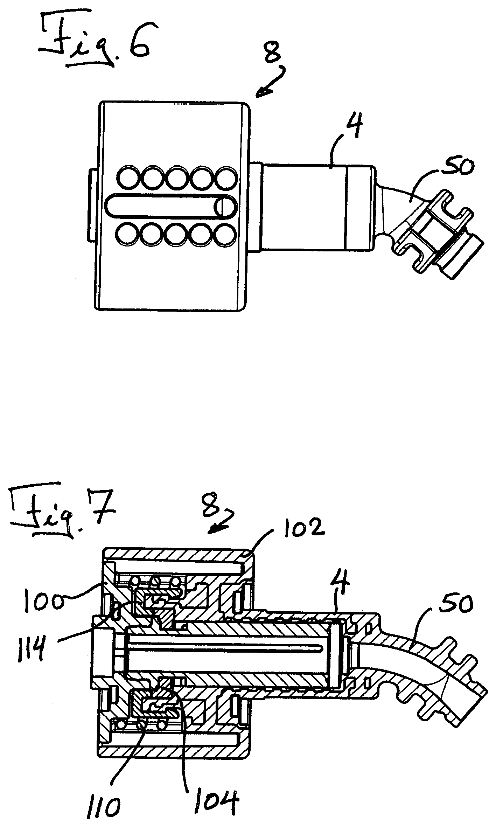

Der Führungsring 104 ist in perspektivischer Darstellung in Figur 10 gezeigt. Dieser Führungsring weist vier Fortsätze 108 auf, die am Innenumfang des Führungsrings 104 angeordnet sind. Die Fortsätze 108 sind, wie hier angedeutet, im Wesentlichen im Querschnitt dreieckig dargestellt, wobei sie jeweils abgeflachte Ecken aufweisen. Die entsprechenden Fortsätze 108 sind versetzt zueinander angeordnet und wirken mit der zackenförmigen Führungsnut 106 derart zusammen, dass sie durch Längsverschiebung der Bauteile 100 und 102 entlang der Führungsnut gleiten und dort in entsprechenden Raststellungen einrasten. Die Raststellungen sind dabei so gewählt, dass die entsprechenden Positionen des Betätigungsmechanismus 1 in . Figur 1 und 2 einstellbar sind. Dabei entspricht die Position gemäß der Figuren 4 und 5 des hier dargestellten Betätigungselementes 8 der Position des Betätigungselementes 8 in Figur 1 und gemäß der Figuren 6 und 7 der Stellung des Betätigungselementes 8 in Figur 2.

Damit der Führungsring 104 mit seinen Fortsätzen 108 in den entsprechenden Rastpositionen der Führungsnut 106 verbleibt, ist eine Feder 110 vorgesehen, die zwischen den Hülsenelementen 100 und 102 angeordnet ist, so dass das Hülsenelement 100 mit der entsprechenden Hülse 9 gegenüber dem Hülsenelement 102 entgegen der Federkraft 110 verschiebbar ist. Dabei ist nach Verlassen der Rastposition, wie sie in den Figuren 4 und 5 dargestellt ist, durch entsprechendes Zusammendrücken der Bauteile 100 und 102 durch Entlanggleiten der Fortsätze 108 entlang der Führungsnut 106 nach Erreichen der Position gemäß der Figuren 6 und 7 eine zweite Rastposition erreicht. Diese Rastposition führt dazu, dass die Hülse 9 in die Führungshülse 4 in der in Figur 7 dargestellten Art und Weise eingeschoben ist.

Durch entsprechenden Druck auf das Bauteil 100 entgegen der Federkraft wird die Rastposition wieder verlassen und durch entsprechendes weiteres Abgleiten der Fortsätze 108 entlang der Führungskulisse 106 wird aufgrund der Kraft der Feder 110 wieder die Rastposition entsprechend Figur 5 erreicht. Aufgrund der Zwangsführung der Führungskulisse 106 und der entsprechend ihrer Form ausgestalteten Fortsätze 108 rotiert während der Längsbewegung der Hülse 9 in der Führungshülse 4 der Führungsring 104 entlang der Führungskulisse 106.

Durch den Wechsel des im wesentlichen aus den Hülsen bestehenden Betätigungselementes 8 oder Aktivierungsteils zwischen den beiden Rastpositionen wird mittels des Seiles 51 das Aktivierungsteil, d. h. hier die Aufnahme 25 mit der die Körperröhre umfassenden Faszie, betätigt.

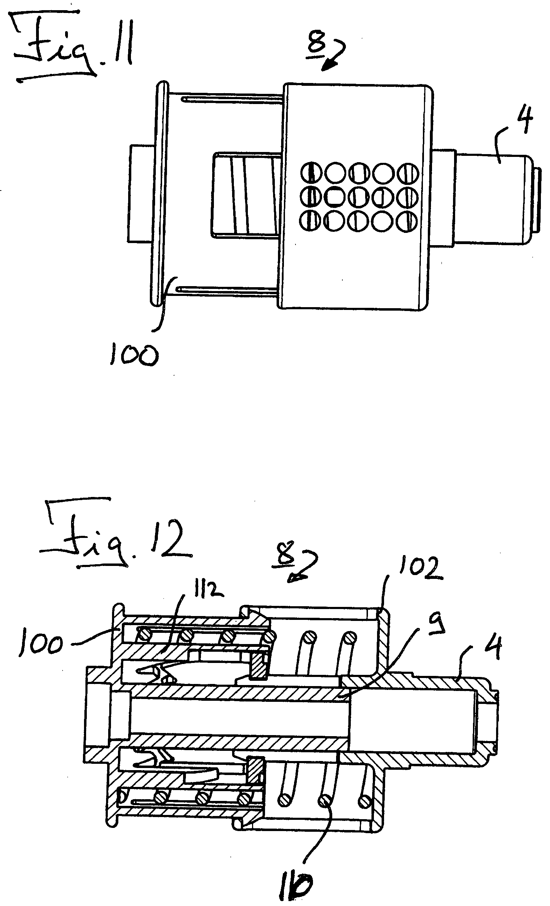

Die anhand der Figuren 11 bis 17 dargestellte Ausführungsvariante des Betätigungselementes 8 entspricht in seiner Funktion wiederum dem Betätigungselement 8 nach der zuvor beschriebenen Ausführungsvariante. Gleiche Teile sind hier mit gleichen Bezugsziffern versehen.

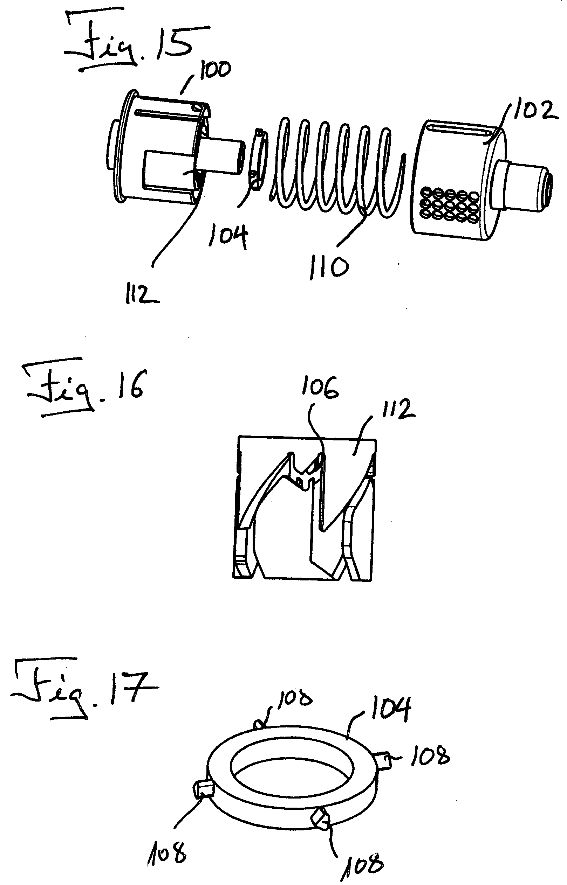

Der wesentliche Unterschied dieser Ausführungsvariante gegenüber der zuvor anhand der Figuren 4 bis 10 erläuterten Ausführungsvariante besteht darin, dass die Führungskulisse 106 in einer parallel zwischen der Hülse 9 und dem diese umgebenden topfförmigen Hülsenelement 100 angeordneten Zwischenhülse 112 ausgenommen ist. Diese ist im hier dargestellten Ausführungsbeispiel einstückig mit dem topfförmigen Hülsenelement 100 ausgebildet. In der Zwischenhülse 112 ist, wie in der Figur 16 dargestellt ist, die Führungskulisse 106 angeordnet. In der Führungskulisse 106 läuft der mit der Führungshülse 4 verbundene Führungsring 104, der ähnliche Fortsätze 108 aufweist, wie der in Figur 10 dargestellte Führungsring. Wie ein Vergleich der Führungsringe 104 nach der Figur 17 der Figur 10 ergibt, ist in der Ausführungsvariante gemäß Figur 10 ein Führungsring 104 dargestellt, dessen Fortsätze 108 zum Inneren des Führungsrings liegen, während dies in der Ausführungsform gemäß Figur 17 ein Führungsring ist, dessen Fortsätze 108 nach außen gerichtet sind. Die Form und Anordnung der Fortsätze 108 ist im Wesentlichen gleich wie in der zuvor beschriebenen Ausführungsform gemäß Figur 10.

Hinsichtlich der Funktionsweise der weiteren Elemente des Betätigungselementes 8 gemäß der Ausführungsform nach den Figuren 11 bis 17 kann auf die vorherige Beschreibung der ersten Ausführungsform nach den Figuren 4 bis 10 verwiesen werden.

## Patentansprüche

1. Implantierbarer, zwischen zwei stabilen Stellungen schaltbarer Betätigungsmechanismus (1) für ein zwischen Zuständen oder Stellungen schaltbares Implantat, mit einem Stützelement (2), das eine Montageplatte (3) zur Abstützung an einem Knochen aufweist und mit einem relativ zum Stützelement (2) bewegbaren, federbeaufschlagten und eine Führungskulisse (106) aufweisenden Betätigungselement (8), das sich durch das Stützelement (2) erstreckt und mittels eines Seiles (51) mit einem einen Wechsel zwischen den beiden Stellungen des Implantates auslösenden, jenseits des Stützelementes (2) vorgesehenen Aktivierungsteil verbunden ist,
**dadurch gekennzeichnet,**
**dass** das federbeaufschlagte Betätigungselement (8) einen Führungsring (104) mit mehr als einem Fortsatz (108) aufweist, wobei die Fortsätze derart in der Führungskulisse (106) geführt sind, dass sie unter Federlast in zwei die vorgenannten Stellungen definierenden Rastpositionen einrastbar sind, wobei der Führungsring (104) mit einer Führungshülse (4) des Betätigungselementes (8) verbunden ist.

2. Betätigungsmechanismus nach Anspruch 1, **dadurch gekennzeichnet, dass** die Führungskulisse (106) in einer inneren Hülse (9) des Betätigungselementes oder in einer eigenen mit dieser verbundenen Zwischenhülse (112) vorgesehen ist.

3. Betätigungsmechanismus nach einem der Ansprüche 1 oder 2, **dadurch gekennzeichnet, dass** die Fortsätze (108) auf der Innenseite des Führungsrings (104) angeordnet sind.

4. Betätigungsmechanismus nach einem der Ansprüche 1 oder 2, **dadurch gekennzeichnet, dass** die Fortsätze (108) auf der Außenseite des Führungsrings (104) angeordnet sind.

5. Betätigungsmechanismus nach einem der Ansprüche 1 - 4, **dadurch gekennzeichnet, dass** die Fortsätze (108) einen im wesentlichen dreieckigen Querschnitt aufweisen.

6. Betätigungsmechanismus nach einem der Ansprüche 1 - 5, **dadurch gekennzeichnet, dass** der Führungsring (4) über ein auf die Führungshülse (104) aufschnappbares Schnappelement (114) längsverschieblich auf der Führungshülse (4) montierbar ist.

7. Betätigungsmechanismus nach einem der Ansprüche 1 - 6, **dadurch gekennzeichnet, dass** der Führungsring (104) vier Fortsätze aufweist.

8. Betätigungsmechanismus nach Anspruch 1, **dadurch gekennzeichnet, dass** die vier Fortsätze (108) auf Umfang des Führungsringes (104) gleichverteilt angeordnet sind.

## Claims

1. An implantable actuation mechanism (1), which can be switched between two stable positions, for an implant which can be switched between states or positions, having a support element (2) which has an assembly plate (3) for support at a bone and having a spring-loaded actuation element (8) which is movable relative to the support element (2), has a guide link (106), extends through the support element (2) and is connected by means of a cable (51) to an activation part initiating a change between the two positions of the implant and provided at the other side of the support element (2),
**characterized in that**
the spring-loaded actuation element (8) has a guide ring (104) having more than one prolongation (108), wherein the prolongations are guided in the guide link (106) such that they can latch into two latch positions defining the aforesaid positions under a spring load, wherein the guide ring (104) is connected to a guide sleeve (4) of the actuation element (8).

2. An actuation mechanism in accordance with claim 1, **characterized in that** the guide link (106) is provided in an inner sleeve (9) of the actuation element or in a separate intermediate sleeve (112) connected thereto.

3. An actuation mechanism in accordance with claim 1 or 2, **characterized in that** the prolongations (108) are arranged on the inner side of the guide ring (104).

4. An actuation mechanism in accordance with claim 1 or 2, **characterized in that**
the prolongations (108) are arranged on the outer side of the guide ring (104).

5. An actuation mechanism in accordance with one of the claims 1 to 4,
**characterized in that** the prolongations (108) have a substantially triangular cross-section.

6. An actuation mechanism in accordance with one of the claims 1 to 5,
**characterized in that** the guide ring (4) can be installed longitudinally displaceably on the guide sleeve (4) via a snap-in element (114) which can snap onto the guide sleeve (104).

7. An actuation mechanism in accordance with one of the claims 1 to 6,
**characterized in that** the guide ring (104) has four prolongations.

8. An actuation mechanism in accordance with claim 1, **characterized in that** the four prolongations (108) are arranged equally distributed over the periphery of the guide ring (104).

## Revendications

1. Mécanisme d'actionnement (1) implantable et commutable entre deux positions stables, destiné à un implant commutable entre différents états ou positions, comprenant un élément de support (2), qui comporte une plaque de montage (3) destinée au support sur un os et comprenant un élément d'actionnement (8) à ressort, mobile par rapport à l'élément de support (2) et doté d'une coulisse de guidage (106), l'élément d'actionnement s'étendant à travers l'élément de support (2) et étant relié au moyen d'un câble (51) à une pièce d'activation qui est prévue au-delà de l'élément de support (2) et qui déclenche une transition entre les deux positions de l'implant,
**caractérisé en ce que**
l'élément d'actionnement (8) à ressort comporte une bague de guidage (104) dotée de plusieurs saillies (108), les saillies étant guidées de telle manière dans la coulisse de guidage (106) qu'elles peuvent, sous la charge du ressort, s'encliqueter dans deux positions d'encliquetage définissant les positions susmentionnées, la bague de guidage (104) étant reliée à une douille de guidage (4) de l'élément d'actionnement (8).

2. Mécanisme d'actionnement selon la revendication 1, **caractérisé en ce que** la coulisse de guidage (106) est prévue dans une douille intérieure (9) de l'élément d'actionnement ou dans une douille intermédiaire (112) propre, reliée à celle-ci.

3. Mécanisme d'actionnement selon l'une des revendications 1 et 2, **caractérisé en ce que** les saillies (108) sont disposées sur la face intérieure de la bague de guidage (104).

4. Mécanisme d'actionnement selon l'une des revendications 1 et 2, **caractérisé en ce que** les saillies (108) sont disposées sur la face extérieure de la bague de guidage (104).

5. Mécanisme d'actionnement selon l'une des revendications 1 à 4, **caractérisé en ce que** les saillies (108) présentent une section transversale sensiblement triangulaire.

6. Mécanisme d'actionnement selon l'une des revendications 1 à 5, **caractérisé en ce que** la bague de guidage (4) peut être montée sur la douille de guidage (4) de manière mobile dans le sens longitudinal par le biais d'un élément à déclic (114) se fixant sur la douille de guidage (104).

7. Mécanisme d'actionnement selon l'une des revendications 1 à 6, **caractérisé en ce que** la bague de guidage (104) comporte quatre saillies.

8. Mécanisme d'actionnement selon la revendication 1, **caractérisé en ce que** les quatre saillies (108) sont réparties de manière homogène sur la périphérie de la bague de guidage (104).
